# EUROPEAN PATENT APPLICATION

(11) **EP 3 456 248 A1**
(43) Date of publication of application: **20.03.2019**
(21) Application number: 17191024.3
(22) Date of filing: 14.09.2017
(51) Int. Cl.: A61B 5/02, A61B 5/026

(54) **HEMODYNAMIC PARAMETERS FOR CO-REGISTRATION**

(71) Applicant: Koninklijke Philips N.V., 5656 AE Eindhoven (NL)
(72) Inventor: HAASE, Christian, 5656 AE Eindhoven (NL); GRASS, Michael, 5656 AE Eindhoven (NL); VAN LAVIEREN, Martijn Anne, 5656 AE Eindhoven (NL)
(74) Representative: de Haan, Poul Erik

(57) **Abstract**

An apparatus for analyzing a vasculature of a patient and a corresponding method are provided in which a plurality of simulated hemodynamic parameter values obtained from (non-invasively) acquired diagnostic images (10) are compared (400) to at least one intravascular hemodynamic parameter value (20) acquired during an invasive measurement in a vessel of interest in the vasculature. The comparison allows to uniquely determine the vessel of interest. Based on this information, the assessment of the disease and the potential treatment planning may be improved.

## Description

### FIELD OF THE INVENTION

The present invention relates to an apparatus for analyzing the vasculature of a patient, a corresponding method and a respective computer program. In particular, the present invention relates to an apparatus for an improved analysis of a vessel of interest in the coronary vasculature employing a comparison of simulated hemodynamic parameter values derived from a physiological model of the vasculature with intravascular hemodynamic parameter values acquired in-situ from the vessel of interest.

### BACKGROUND OF THE INVENTION

A correct assessment of a coronary disease is of great importance for determining optimized treatment options. This assessment requires precise knowledge about the patient's coronary vasculature, both in terms of geometry and in terms of hemodynamic parameters, i.e. the characteristics of the blood in said vasculature. This knowledge is typically achieved by obtaining the required information about the patient's vasculature, such as geometry of the vasculature, hemodynamic parameter values at certain points in the vasculature and indices that are related to said hemodynamic parameter values. This multitude of different information may hereby be obtained using a combination of different measurement modalities.

Typically, the geometry of the vasculature may be derived from diagnostic image data that is acquired using non-invasive medical imaging modalities, such as computed tomography (CT), magnetic resonance imaging (MRI), modeling from multiple X-ray projections or ultrasound imaging. The geometry of the vasculature may particularly be represented in a three-dimensional reconstruction of the vasculature reconstructed from the diagnostic image data.

The hemodynamic parameter values and their related indices, such as fractional flow reserve (FFR) or instantaneous Wave-Free Ratio (iFR), are usually determined by means of an invasive measurement modality in which a measurement device, usually comprising a sensor equipped guide wire, is introduced into a vessel of interest and one or more values for at least one hemodynamic parameter are obtained at one or more intravascular positions inside the vessel of interest.

In order to achieve thorough understanding of the vasculature, the two modalities have to be co-registered. That is, it has to be determined which position in the reconstruction corresponds to a respective intravascular position of the invasive measurement. Co-registration may be achieved by acquiring one or more tracking images of the measurement device introduced into the vessel of interest. These tracking images may for example be acquired using fluoroscopy imaging. The position of the intravascular measurement device, in particular, the position of a tip of the intravascular measurement device, is then determined based on one or more acquired fluoroscopic tracking images.

The thus acquired tracking images are then co-registered with the reconstruction from the diagnostic image data. That is, a position of the measurement device in the tracking image is identified. Since the measurement device is introduced into the vessel of interest, the identifying of the position of the measurement device means an identifying of a position in the vessel of interest in the tracking image. Subsequently, it is determined which vessels in the reconstruction are located such that their positions in the reconstruction may correspond to the position in the vessel of interest in the tracking image. The thus determined vessel is designated the vessel of interest in the reconstruction. Thus, by correlating the positions in the tracking image and in the reconstruction the vessel of interest may be identified.

### SUMMARY OF THE INVENTION

In known co-registration approaches, the reconstruction is typically a three-dimensional representation of the vasculature or the vessel of interest. The at least one tracking image, however, is usually a two-dimensional representation. Accordingly, when co-registering a three-dimensional representation to a two-dimensional representation, there is a certain ambiguity in this correlation. This ambiguity may be caused by the overlapping of multiple vessels in the two-dimensional representation or by cardiac motion, which may cause the vessel of interest to appear at different locations during the cardiac cycle or like effects.

The vessel of interest in the two-dimensional tracking image may therefore be found correspond to two (or more) vessels in the three-dimensional reconstruction. In this case, it is not possible to uniquely identify the location of the vessel of interest in the reconstruction. This may lead to an incorrect assessment of the vasculature and to an incorrect determination of the patient's health status and the potential treatment options.

It is therefore an object of the present invention to provide an improved apparatus and a corresponding method for analyzing the coronary vasculature of a patient.

It is a further object of the invention to provide an apparatus for analyzing the coronary vasculature of a patient, which allows a correct identification of the vessel of interest in the reconstruction of the vasculature derived from diagnostic image data. More particularly, it is an object of the invention to provide an apparatus for a unique correlation of at least one intravascular position at which the at least one intravascular hemodynamic parameter value has been acquired to a corresponding position in the reconstruction.

Thus, an apparatus is provided for analyzing a vasculature of a patient which comprises an input unit configured to receive a plurality of simulated hemodynamic parameter values determined on the basis of a physiological model derived from diagnostic image data of the vasculature for a plurality of candidate vessels of the vasculature and for receiving at least one intravascular hemodynamic parameter value acquired in-situ by an intravascular measurement device from a vessel of interest in the vasculature. The apparatus further comprises a comparing unit configured to compare each of the plurality of simulated hemodynamic parameter values to the at least one intravascular hemodynamic parameter value and to identify, based on the comparing, the vessel of interest in a reconstruction derived from the diagnostic image data.

In this context, the term hemodynamic parameter may be considered as referring to a characteristic of the blood in the vasculature, such as the blood flow, blood resistance, blood pressure or the like. The term hemodynamic parameter value may refer to a value of the hemodynamic parameter at a particular position of the vessel, i.e. the magnitude of that particular hemodynamic parameter that is measured for the particular position. The term hemodynamic parameter value may further refer to an index for a hemodynamic parameter, such as an FFR or iFR value.

Hereby, at least one hemodynamic parameter value may particularly be determined for at least one position in the vessel of interest. This determination may be performed by calculating a hemodynamic parameter value for a particular position on the basis of a physiological model. In this case the hemodynamic parameter value is referred to as a simulated hemodynamic parameter value.

Else, the determination may be performed by measuring the hemodynamic parameter value at a particular intravascular position in an invasive procedure. In this case, the hemodynamic parameter value is referred to as an intravascular hemodynamic parameter value.

The intravascular hemodynamic parameter value may particularly be obtained using an intravascular measurement device. In this context, the intravascular measurement device may particularly be a sensor equipped measurement wire that may be introduced into the vessel of interest and may acquire one or more intravascular hemodynamic parameter values from inside the vessel of interest.

The term diagnostic image data may particularly refer to a plurality of diagnostic projection images. These diagnostic projection images may hereby particularly refer to images representing the coronary vasculature. More particularly, the diagnostic projection images may represent the coronary vasculature including a vessel of interest. The plurality of diagnostic projection images may particularly be obtained by measuring the vasculature by means of a non-invasive diagnostic imaging modality. Examples for such non-invasive diagnostic imaging modalities refer to X-ray based techniques, such as X-ray scanning, CT, position emission tomography (PET), single position emission computed tomography (SPECT) or by techniques such as MRI or ultrasound imaging, as well as combinations thereof.

The non-invasive diagnostic imaging modality may be gated. Such gated non-invasive diagnostic imaging modalities may typically employ a gated reconstruction, in which the acquisition of the diagnostic projection images is performed in parallel with acquisition of data providing information over the cardiac cycle, such as electrocardiogram (ECG) or photoplethysmographic (PPG) data. This data is hereby used to gate the image acquisition and the reconstruction by means of respectively selected phase points of the cardiac cycle.

Accordingly, the diagnostic projection images may be used by a respective reconstruction unit to provide a reconstruction of the vasculature. The reconstruction of the vasculature may refer to a two-dimensional or three-dimensional reconstruction of the vasculature. More specifically, the reconstruction may refer to a three-dimensional (3D) reconstruction from the diagnostic image data. The reconstruction is provided to the input unit from the reconstruction unit.

In this context, the term physiological model refers to a computed model of the vasculature, which is generated from at least one diagnostic projection image of the diagnostic image data. The generating of the physiological model may comprise a segmentation of one or more vessels in the imaged vasculature. It shall be understood that the segmentation of the one or more vessels refers to a segmentation of the one or more vessels as represented in the diagnostic image data, i.e. as represented by one or more diagnostic projection images in the diagnostic image data. Likewise, the segmentation may refer to a segmentation of the one or more vessels as represented in the reconstruction of the diagnostic image data. Thus, the deriving of the physiological model on the basis of the diagnostic image data may refer to a deriving of the physiological model from one or more of the diagnostic projection images in the diagnostic image data or to a deriving from the 3D reconstruction of the diagnostic image data.

Based on the segmentation, a physiological model may be generated representing one or more vessel segments of the one or more vessels in the vasculature.

The physiological model may particularly be used to simulate the fluid dynamics through the vessels in the vasculature. That is, the physiological model may be said to represent the blood flow through the patient's vasculature using a respective fluid dynamics simulation, also called a fluid dynamics model.

The physiological model may particularly be generated by performing calculations that simulate the interaction of the blood with the vessel wall, i.e. the inner surface of the vessel through which the blood is flowing. These interactions are defined by certain boundary conditions that take account of the properties of the vessel, such as vessel wall composition, vessel wall elasticity and vessel impedance, bifurcations in the vessel as well as properties of the blood, such as blood viscosity.

The physiological model may particularly be implemented as a lumped parameter fluid dynamics model. In such a lumped parameter fluid dynamics model, the fluid dynamics of the vessels are approximated by a topology of discrete entities. More particularly, in the lumped parameter fluid dynamics model, the vessel impedance is approximated by a series of resistor elements while the termination of a vessel is represented by an element representing ground. Thus, a vasculature including multiple vessels in a vessel tree may be represented by a topology of resistors each having a particular resistance with the representation of the vasculature being terminated by respective ground elements. These lumped parameter models reduce the number of dimensions compared to other approaches such as Navier-Stokes or the like. Accordingly, using a lumped parameter model may allow for a simplified calculation of the fluid dynamics inside the vessels and may ultimately result in reduced processing time. The employing of such a lumped parameter model is described for example in international application WO 2016/087396.

In order to (further) reduce the complexity of the model, generalized boundary conditions may hereby be used. These generalized boundary conditions may be similar for all patients or for particular groups of patients, which may be distinguished by age, gender, physiological condition or the like. Likewise, patient-specific boundary conditions that have been derived for a particular patient may be used for calculation of the physiological model.

In this context, the term candidate vessel refers to a vessel represented in the reconstruction of the vasculature which is a potential candidate for being the vessel of interest. In accordance with the invention, a plurality of candidate vessels may be determined in the 3D reconstruction. In particular, the plurality of candidate vessels may be derived on the basis of one or more tracking images representing the vessel of interest. Likewise, the plurality of candidate vessels may be derived on the basis of the geometry of the vessel of interest as obtained from the invasive measurement, for example by regarding a pullback curve obtained from an invasive pullback recording through the vessel of interest.

Each of the plurality of candidate vessels may be identified because its position and/or geometry as represented by the reconstruction correspond to that of the vessel of interest as visualized using the tracking image, the pullback curve, or the like. As such, the plurality of candidate vessels defines a subset of the vessels in the vasculature which may potentially correspond to the vessel of interest.

In some embodiments, for each candidate vessel, at least one simulated hemodynamic parameter value may be determined at least one position in the candidate vessel. More particularly, the in-situ measurement may be performed to acquire a single intravascular hemodynamic parameter value at a specific intravascular position inside the vessel of interest. In this case, for each candidate vessel, it may be determined which position inside the candidate vessel would correspond to the specific intravascular position in the vessel of interest, if the candidate vessel were the vessel of interest. Subsequently, for each candidate vessel, a single simulated hemodynamic parameter value may be determined at that particular position inside the candidate vessel.

The in-situ measurement may further be performed to record a series intravascular hemodynamic parameter values at a plurality of intravascular positions along a longitudinal axis of the vessel of interest. Hereby, the in-situ measurement may particularly be performed by pullback recording of at least one hemodynamic parameter value during pullback of a measurement device along the longitudinal axis of the vessel of interest. During this pullback, at least one intravascular hemodynamic parameter value may be acquired for each intravascular position along a certain pullback length.

In this case, a plurality of simulated hemodynamic parameter values, i.e. a respective series of simulated hemodynamic parameter values, may be determined for each candidate vessel. Each of the series of simulated hemodynamic parameter values is determined at a respective position corresponding to an intravascular position of the invasive measurement. Subsequently, the series of intravascular hemodynamic parameter values may be compared to each series of the simulated hemodynamic parameter values for each of the candidate vessels. In this case, the simulation may in particular encompass the modeling of a simulated pullback curve along a (virtual) longitudinal axis of each of the candidate vessels.

Further, a series of intravascular hemodynamic parameter values may be acquired at a plurality of intravascular positions along the longitudinal axis of the vessel of interest, but only a single simulated hemodynamic parameter value may be determined for each candidate vessel. In this case, an intravascular hemodynamic parameter value acquired at a particular intravascular position may be selected. Subsequently, the corresponding positions in each of the candidate vessels may be derived. Then, for each of the candidate vessels, the simulated hemodynamic parameter value may be determined for the respective corresponding position.

In an embodiment, each of the plurality of simulated parameter values may be compared to the at least one intravascular hemodynamic parameter value. This comparing may be used to identify the vessel of interest in the plurality of candidate vessels represented by the reconstruction. More specifically, the vessel of interest in the reconstruction may be identified by considering for which candidate vessel the fluid dynamics are most similar to that of the invasively measured vessel of interest.

As such, this approach allows to use the fluid dynamics as an additional similarity measure to correctly identify a vessel of interest in a reconstruction of the vasculature.

More particularly, a two-step co-registration approach is foreseen which allows to forego the ambiguity of the co-registration according to the prior art: In a first step, a geometry- or image-based correlation of the invasively acquired measurement data to the reconstruction derived from the non-invasively obtained diagnostic image data is performed to identify one or more candidate vessels for the vessel of interest. In a second step, a fluid dynamics model is used to determine simulated hemodynamic parameter values for each of the candidate vessels and these simulated hemodynamic parameter values are then used to uniquely determine the vessel of interest based on a similarity of the simulated hemodynamic parameter values for one particular candidate vessel and the intravascular hemodynamic parameter values of the vessel of interest.

According to an embodiment, the diagnostic image data comprises CT projection data; and the reconstruction comprises volumetric data reconstructed from the computed tomography projection data.

In some embodiments, the diagnostic image data may be obtained using computed tomography imaging. Thus, the diagnostic image data may comprise respective CT projection data of the vasculature. In the context of the embodiment, CT projection data refers to a set of diagnostic projection images of the vasculature obtained for a number of different projection angles. The CT projection data thus may be considered a three-dimensional image data set, which can be used to reconstruct volumetric data of the vasculature, i.e. to obtain a three-dimensional reconstruction of the vasculature. Various methods and apparatuses for providing said volumetric data from CT projection data are known to the skilled person and may e.g. be found in WO 2004/037087 or EP 0989520.

In accordance with a further embodiment the at least one intravascular hemodynamic parameter value is acquired using the intravascular measurement device for pullback recording.

In some embodiments, a pullback recording may be used to acquire at least one intravascular hemodynamic parameter value. More particularly, a pullback recording may be used to acquire a plurality of intravascular hemodynamic parameter values along a longitudinal axis of the vessel of interest. The thus obtained intravascular hemodynamic parameter values may be represented as a pullback curve, i.e. a representation of the intravascular hemodynamic parameter value as a function of the pullback length inside the vessel of interest. The acquisition of a plurality of intravascular hemodynamic parameter values by means of a pullback recording may particularly allow to estimate the vessel length and the progression of the vessel of interest in the vasculature.

This may allow to perform the co-registration directly, based on the further information obtained from the pullback recording, thereby avoiding the necessity to acquire one or more additional tracking images. Thus, by using a pullback recording to obtain the intravascular hemodynamic parameter value, the use of further imaging modalities may be avoided.

According to an even further embodiment, the apparatus further comprises a modeling unit configured to generate the physiological model based on the diagnostic image data of the vasculature, the physiological model representing the fluid dynamics through the plurality of candidate vessels and a determination unit configured to determine the plurality of simulated hemodynamic parameter values on the basis of the physiological model. In some embodiments, the apparatus further comprises a modeling unit which receives the diagnostic image data of the vasculature as acquired by the imaging modality and generates the physiological model on the basis of the diagnostic image data. More particularly, the modeling unit may be configured to segment one or more vessels of the imaged vasculature as represented by the diagnostic projection images of the diagnostic image data. The modeling unit may then, based on the segmentation, model the fluid dynamics of the fluid flow, i.e. the blood flow, through the segmented vessels in the vasculature. That is, the modeling unit may provide a physiological model representing the fluid dynamics through one or more vessels of the vasculature imaged by the imaging modality. The physiological model may then be used to approximate the fluid dynamics.

Even more specifically, the physiological model may be used by a determination unit to estimate the fluid dynamics through each of the candidate vessels to determine the simulated hemodynamic parameter value or the plurality of simulated hemodynamic parameter values for each candidate vessel. That is, the determination unit may calculate at least one hemodynamic parameter value at least one position in each candidate vessel, wherein the at least one position in the candidate vessel has been derived to correspond to a respective intravascular position at which the intravascular hemodynamic parameter value has been acquired from the vessel of interest. The determination unit may then provide the thus determined plurality of simulated hemodynamic parameter values for the candidate vessels to the input unit for further processing.

Accordingly, in a further embodiment the determining the plurality of simulated hemodynamic parameter values comprises determining, for each of the candidate vessels, a respective simulated hemodynamic parameter value and the identifying the vessel of interest comprises identifying the candidate vessel for which the respective simulated hemodynamic parameter exhibits the best agreement with the at least one intravascular hemodynamic parameter.

In some embodiments, the identifying of the vessel of interest may be performed by determining the candidate vessel for which the fluid dynamics are the most similar to the fluid dynamics of the vessel of interest as determined from the invasive measurement. More particularly, the at least one simulated hemodynamic parameter value derived for each of the candidate vessels may be compared to the at least one intravascular hemodynamic parameter value in the vessel of interest to determine for which candidate vessel the at least one simulated hemodynamic parameter values is the best match for the intravascular hemodynamic parameter value, i.e. shows the highest correspondence with the intravascular hemodynamic parameter value.

According to yet another embodiment, the input unit is configured to receive at least one additional information indicative of the vessel of interest. The comparing each of the plurality of simulated hemodynamic parameter values to the at least one intravascular hemodynamic parameter value comprises co-registering the at least one intravascular hemodynamic parameter to the reconstruction of the diagnostic image data based on the at least one additional information and a deriving, based on the co-registering, the plurality of candidate vessels.

In some embodiment, the candidate vessels in the reconstruction may be identified using additional information that indicates the identity of the vessel of interest. This allows to register the at least one intravascular hemodynamic parameter value to the reconstruction.

In this context, the additional information may particularly refer to tracking information that has been obtained for the measurement device. More particularly, such tracking information may be obtained by tracking the position of the measurement device during the intravascular measurement. Hereby, such tracking may particularly be performed using electromagnetic or fiber-optic catheter tracking.

The additional information may also refer to additional measurement parameters, such as the projection angle of the diagnostic imaging modality or the pullback length or pullback duration of the in-situ measurement.

Using the information about the projection angle for which one particular diagnostic projection image has been obtained may allow identifying the vessel which is being examined by the diagnostic image data. Even more specifically, the measurement angle of the C-arm of an X-ray unit may be used to derive the vessel of interest from the respective diagnostic image data.

Further, the duration or the length of the pullback of the intravascular measurement device may be used to derive further geometric information about the vessel of interest, thereby allowing the identification of the vessel of interest.

Likewise, the additional information may refer to patient parameters, in particular parameters indicative of the cardiac motion of the patient, such as ECG data or PPG data. Further, the patient parameters may also refer to a delay of the hemodynamic parameter value, in particular the pressure delay, relative to the determined ECG. These patient parameters particularly allow alleviating the ambiguity due to cardiac motion.

In some embodiments, the different imaging and measurement modalities used to achieve the unique co-registration may conveniently be implemented in a picture archiving and communication system (PACS), which provides storage capabilities for and access to respective image data that has been obtained by different imaging modalities.

According to an even further embodiment the input unit is further configured to receive at least one tracking image of the measurement device and the comparing each of the plurality of simulated hemodynamic parameters to the at least one intravascular hemodynamic parameter comprises a co-registering the at least one tracking image to the reconstruction of the diagnostic image data and a deriving, based on the co-registering, the plurality of candidate vessels.

In some embodiments the measurement data alone may not suffice to identify potential candidate vessels in the reconstruction of the vasculature. Thus, it may be necessary to use one or more tracking images of the measurement device introduced into the vessel of interest. From these tracking images, further information about the geometry and/or progression or the like of the vessel of interest may be derived. This allows identifying the candidate vessels in the reconstruction.

The at least one tracking image may particularly refer to a two-dimensional (2D) tracking image of the measurement device inside the vessel of interest. The 2D tracking image may particularly be co-registered to a 3D reconstruction from the diagnostic image data using a geometry- based or image-based co-registration of the respective coordinate systems. Hereby, the at least one tracking image may for example be acquired using a fluoroscopy imaging modality.

In some embodiments, the co-registering of the at least one intravascular hemodynamic parameter to the reconstruction of the diagnostic image data may particularly be performed by co-registering at least one tracking image to the reconstruction of the diagnostic image data and by further using the additional information to enhance the accuracy of the image-based co-registration. Likewise, the co-registering of the intravascular hemodynamic parameter to the reconstruction of the diagnostic image data may be performed by first co-registering the at least one intravascular hemodynamic parameter to the reconstruction and subsequently using the image-based co-registration to support the co-registering process, i.e. to improve the accuracy of the first-step co-registering.

In accordance with yet another embodiment, the measurement device is configured to acquire the at least one hemodynamic parameter value at a particular intravascular position in the vessel of interest. The deriving the plurality of candidate vessels comprises identifying, for each of the candidate vessels, a respective candidate position corresponding to the intravascular position. The determining the simulated hemodynamic parameter values comprises determining, for each of the candidate vessels, a respective simulated hemodynamic parameter value at the respective candidate position and the comparing comprises comparing the at least one intravascular hemodynamic parameter value acquired at the intravascular position to each of the simulated hemodynamic parameter values determined at the respective candidate positions. The identifying the vessel of interest then comprises identifying the candidate position for which the respective simulated hemodynamic parameter value exhibits the best agreement with the at least one intravascular hemodynamic parameter value.

In some embodiments the measurement device may be used to acquire a single intravascular hemodynamic parameter value at a specific intravascular position in the vessel of interest. In this case, it may be necessary to determine the simulated hemodynamic parameter value at the corresponding position in each of the candidate vessels. This means that, for each candidate vessel, a respective candidate position may be determined which corresponds to the intravascular position of the measurement, if the respective candidate vessel were considered the vessel of interest.

In some embodiments, this determination of candidate positions may particularly be performed using the tracking image. Hereby, a co-registration of the tracking image and the volumetric reconstruction may be used to correlate the representation of the intravascular position in the tracking image with corresponding positions in the candidate vessels shown in the reconstruction. These corresponding positions may then be considered the candidate positions at which the simulated hemodynamic parameter value may be calculated.

The physiological model may then be used to determine at least one respective simulated hemodynamic parameter value at each candidate position. Thus, the physiological model may be used to determine, for each candidate vessel, a simulated hemodynamic parameter value at one particular candidate position. Subsequently, the simulated hemodynamic parameter value for each candidate position may be compared to the single intravascular hemodynamic parameter value acquired at the specific intravascular position. Hereby, the simulated hemodynamic parameter value is selected that most closely matches the intravascular hemodynamic parameter value. The candidate vessel for which candidate position the selected simulated hemodynamic parameter value has been determined is then considered to correspond to the vessel of interest.

In accordance with a further embodiment, the apparatus comprises a calculation unit configured to calculate a first graphical representation of the at least one intravascular hemodynamic parameter value and a second graphical representation of the reconstruction of the diagnostic image data, and a display unit configured to jointly display the first representation and the second representation. According to yet another embodiment, the jointly displaying the first graphical representation and the second graphical representation comprises an annotating the first graphical representation to the second graphical representation.

In some embodiments, a graphical representation of the co-registered reconstruction and the at least one intravascular hemodynamic parameter may be provided. More particularly, a first graphical representation for the at least one intravascular hemodynamic parameter and a second graphical representation for the reconstruction may be calculated by a calculation unit and then provided to the display unit. In this context, the display unit may particularly be a computer screen, such as a touch screen. The display unit may then jointly display the first and second graphical representation.

Hereby, the display unit may be used to annotate one or more respective intravascular hemodynamic parameter values to the vessel of interest. That is, the display unit may display the second graphical representation of the reconstruction such that the first graphical representation of a particular intravascular hemodynamic parameter value is illustrated in close proximity to the vessel of interest, particularly in close proximity to a position in the reconstructed vessel of interest which corresponds to the intravascular position at which the intravascular hemodynamic parameter value has been acquired. The first graphical representation may hereby appear inside the vessel of interest in the second graphical representation at the position or may be annotated by means of an arrow pointing at the position.

The joint display of the first graphical representation of at least one intravascular hemodynamic parameter value with the second representation of the volumetric reconstruction allows a user, such as a doctor or nurse, to quickly derive not only the intravascular hemodynamic parameter value, but also the position within the vasculature at which it has been acquired. This eases the assessment of the vasculature and the respective treatment planning.

According to a further aspect of the invention, a method for analyzing a vasculature of a patient is provided. The method comprises the steps of receiving a plurality of simulated hemodynamic parameters determined on the basis of a physiological model derived from diagnostic image data of the vasculature for a plurality of candidate vessels of the vasculature and receiving at least one intravascular hemodynamic parameter acquired in-situ by an intravascular measurement device from a vessel of interest in the vasculature. The method further comprises comparing each of the plurality of simulated hemodynamic parameters to the at least one intravascular hemodynamic parameter and identifying, based on the comparing, the vessel of interest in a reconstruction based on the diagnostic image data.

According to an embodiment, the method further comprises the steps of receiving at least one tracking image of the measurement device, co-registering the at least one tracking image to the reconstruction of the diagnostic image data and deriving, based on the co-registering, the plurality of candidate vessels.

In accordance with yet another embodiment, the method further comprises the steps of calculating a first graphical representation of the at least one intravascular hemodynamic parameter and a second graphical representation of the reconstruction of the diagnostic image data and jointly displaying the first and second representation.

In a further aspect, a computer program for controlling an apparatus according to the invention is provided, which, when executed by a processing unit, is adapted to perform the method according to the invention. In an even further aspect, a computer-readable medium having stored thereon the computer program is provided.

It shall be understood that the apparatus for analyzing a vasculature of a patient may be implemented by means of a processing unit. Hereby, the input unit, the comparing unit, the modeling unit, the determination unit and the calculation unit may be implemented as modules in the processing unit. The functionality of these modules may in particular be implemented by means of a respective algorithm. This algorithm may in particular be implemented using a machine learning algorithm implemented on said processing unit including said modules.

It shall be understood that the apparatus of claim 1, the method of claim 11, the computer program of claim 14, and the computer-readable medium of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a diagnostic imaging modality for obtaining diagnostic image data according to an embodiment.
Fig. 2 schematically illustrates the ambiguity in the co-registration using a back projection of a tracking image to the reconstruction of the vasculature from the prior art.
Fig. 3 schematically illustrates an apparatus for analyzing a patient's vasculature according to an embodiment.
Fig. 4 represents a flow chart for a method for analyzing coronary vessels according to an embodiment.
Fig. 5 schematically illustrates a conclusive determination of a vessel of interest in the reconstruction according to an embodiment.

### DETAILED DESCRIPTION OF EMBODIMENTS

The illustration in the drawings is schematically. In different drawings, similar or identical elements are provided with the same reference numerals.

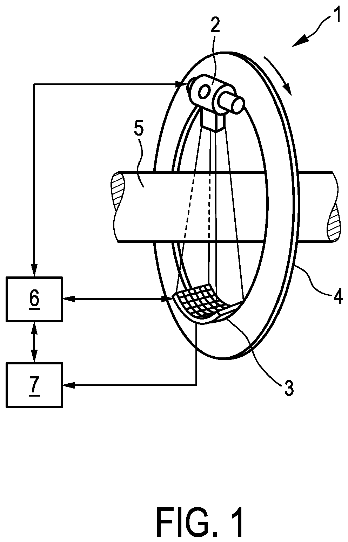

Fig. 1 shows a diagnostic imaging modality for obtaining diagnostic image data. In this particular embodiment, the diagnostic imaging modality corresponds to a computed tomography system 1. The computed tomography system 1 comprises an X-ray source 2, such as an X-ray tube, and an X-ray detector 3, which are mounted opposite to each other on a rotatable gantry 4. The gantry 4 may be configured as annular device, a C-arm, or it may be realized by two robotic arms, for example.

By means of rotating the gantry 4, the X-ray source 2 and the X-ray detector 3 are rotated around an object of interest to be imaged. Hereby, the object of interest is positioned in a measurement region 5 located between the X-ray source 2 and the X-ray detector 3. In the present embodiment, the object of interest is a patient which is assumed to suffer from a coronary artery disease. The computed tomography system 1 is therefore used to image the coronary vasculature of this patient. The computed tomography system 1 is used to obtain diagnostic image data comprising a plurality of diagnostic projection images for different projection angles while rotating the gantry 4 around the patient.

Hereby, the components of the computed tomography system 1 are controlled by means of a control unit 6, which may be configured as a computer-implemented unit comprising a computer program which includes procedures for controlling the components of the computed tomography system 1 and which is executed in a computer device connected to the components of the computed tomography system 1 in a suitable manner.

In order to provide a reconstruction from the diagnostic image data comprising the diagnostic projection images, computed tomography system 1 comprises a reconstruction unit 7. The reconstruction unit 7 may likewise be a computer-implemented unit which comprises a computer program that provides procedures for providing a volumetric reconstruction, i.e. volumetric data, from the diagnostic image data.

In accordance with the invention, the volumetric reconstruction and the diagnostic image data are then provided to an apparatus for analyzing the coronary vasculature by co-registering the volumetric reconstruction to at least one intravascular hemodynamic parameter value that has been acquired in-situ using an intravascular measurement device.

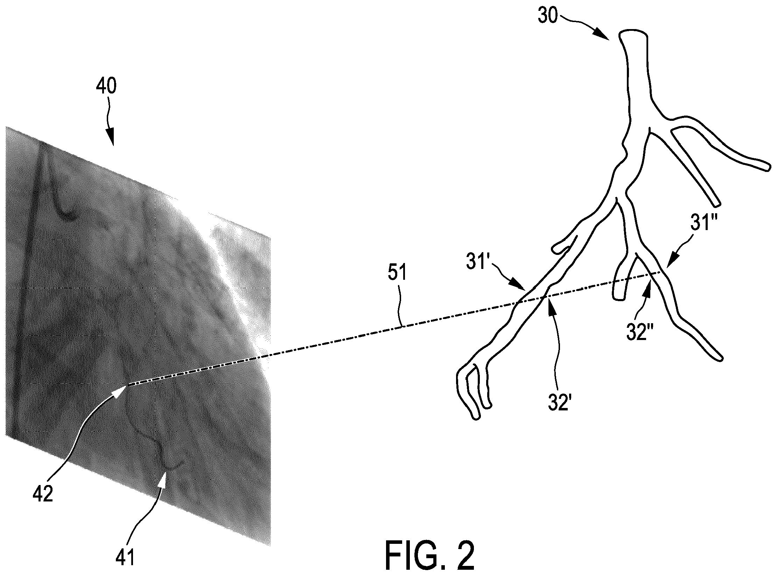

In that respect, Fig. 2 schematically illustrates the ambiguity in the co-registration of the intravascular measurement and a volumetric reconstruction 30 of the vasculature, when the co-registration is based on a back projection of a tracking image 40 to the volumetric reconstruction 30 as known from the prior art.

In the exemplary embodiment of Fig. 2, tracking image 40 has been obtained using X-ray fluoroscopy. The tracking image 40 represents a vessel of interest 41, in which an intravascular measurement device has been introduced to acquire at least one intravascular hemodynamic parameter value.

The tracking image 40 is correlated to the volumetric reconstruction 30 by means of a back projection 51. Thus, the representation of an intravascular measurement position is projected onto a corresponding position in the volumetric reconstruction 30. The back-projection renders two possible candidate positions 32' and 32" in two candidate vessels 31' and 31" in the volumetric reconstruction 30 of the vasculature which may correspond to the intravascular position 42 in the vessel of interest 41 represented in the tracking image 40. From the information provided in Fig. 2, it is not possible to identify which one of the candidate positions 32', 32" is the correct position. Thus, it is not possible to uniquely assign one of the candidate vessels 31', 31" as the vessel of interest in the volumetric reconstruction.

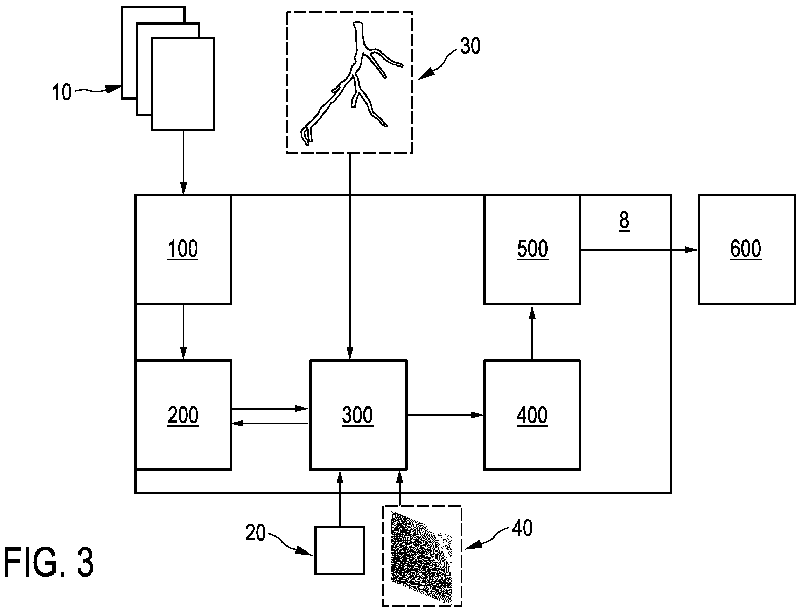

In order to solve this problem, an improved apparatus 8 for analyzing a patient's vasculature according to an exemplary embodiment is illustrated in Fig. 3. Apparatus 8 comprises a modeling unit 100, a determination unit 200, an input unit 300, a comparing unit 400, a calculation unit 500 and a display unit 600.

The modeling unit 100 receives diagnostic image data 10 comprising a plurality of diagnostic projection images. The modeling unit 100 may segment the imaged vessels and generate a physiological model on the basis of this segmentation. The physiological model hereby represents the fluid dynamics through each of the modeled vessels. The modeling unit 100 then provides the physiological model to determination unit 200.

The determination unit 200 receives the physiological model generated based on diagnostic image data 10. Further, the determination unit 200 may optionally receive information regarding a plurality of candidate positions from the input unit 300. The determination unit 200 then uses the physiological model to determine, for each of the candidate vessels 31', 31 ", at least one respective simulated hemodynamic parameter value. In this particular example, the determination unit 200 uses the physiological model to determine, for each of the candidate vessels 31', 31" a respective simulated hemodynamic parameter value at a respective candidate position 32', 32"in the candidate vessel 31', 31".

In this exemplary embodiment, these candidate vessels 31', 31" and their candidate positions 32', 32"have been determined by the input unit 300 based on the tracking image 40. More specifically, the input unit 300 receives the tracking image 40 from a fluoroscopic imaging modality and the volumetric reconstruction 30 from the reconstruction unit 7 of computed tomography system 1. Furthermore, the input unit 300 received an intravascular hemodynamic parameter value 20 from an intravascular measurement device which has been acquired in-situ at a specific intravascular position.

Based on the tracking image 40, the input unit 300 may derive a plurality of candidate vessels 31', 31" in the volumetric reconstruction 30 by co-registering the tracking image 40 and the volumetric reconstruction 30. In the example according to Fig. 3, this is achieved by correlating the two imaging modalities by determining, for each candidate vessel 31', 31" in the volumetric reconstruction 30, a candidate position 32', 32"that may correspond to the intravascular position in the vessel of interest at which the intravascular hemodynamic parameter value 20 has been obtained. This intravascular position is hereby derived from the tracking image 40.

The input unit 300 then provides respective information about the plurality of candidate positions 32', 32" to the determination unit 200, upon which the determination unit 200 determines the plurality of simulated hemodynamic parameter values. In the exemplary embodiment, the determination unit 200 particularly determines a simulated hemodynamic parameter value for each one of the candidate positions 32', 32" as described above and then provides the plurality of simulated hemodynamic parameter values to the input unit 300.

The input unit 300 then provides the plurality of simulated hemodynamic parameters, optionally along with their respective candidate positions 32', 32", as well as the intravascular hemodynamic parameter value 20 and the volumetric reconstruction 30 to the comparing unit 400.

The comparing unit 400 compares each of the simulated hemodynamic parameter values to the intravascular hemodynamic parameter value 20 and identifies the simulated hemodynamic parameter value that shows the highest agreement with the intravascular hemodynamic parameter value 20.

In this manner, the comparing unit 400 identifies the candidate vessel 31', 31" for which the simulated hemodynamic parameter value shows the best agreement to intravascular hemodynamic parameter value 20, which has been obtained in situ in the vessel of interest. Thereby, the comparing unit 400 identifies the vessel of interest in volumetric reconstruction 30. Comparing unit 400 then provides the information about the identified vessel of interest in the volumetric reconstruction 30 along with the volumetric reconstruction 30 and the intravascular hemodynamic parameter value 20 to the calculation unit 500.

Calculation unit 500 calculates a first representation of the intravascular hemodynamic parameter value 20 and a second representation of the volumetric reconstruction 30. Calculation unit 500 then provides the first and second representation to the display unit 600.

Based on the identification of the vessel of interest in the volumetric reconstruction 30 and the intravascular hemodynamic parameter value 20 the display unit 600 then jointly displays the first and the second representation. In the exemplary embodiment according to Fig. 3, the joint display is performed by annotating the first representation to a specific position in the second representation. This specific position may correspond or be proximate to the position indicating the intravascular position in the volumetric reconstruction 30. The annotation may hereby particularly performed by inserting the first representation into the second representation and further inserting a graphical illustration of an indicator, such as an arrow, to indicate the identified position.

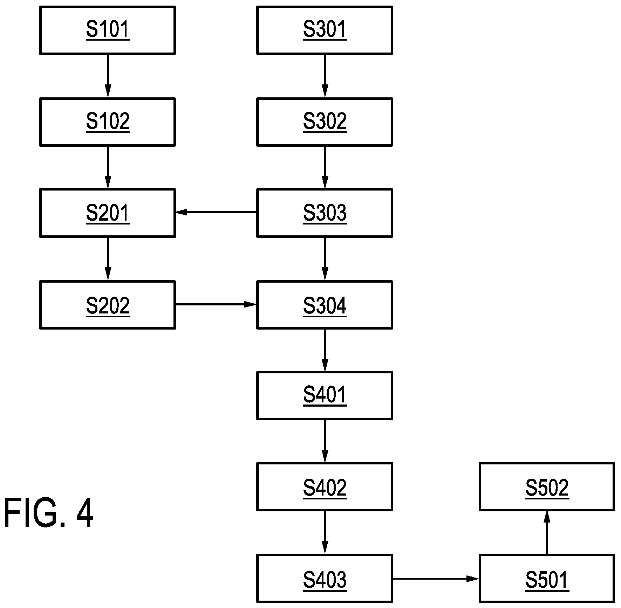

Fig. 4 schematically illustrates a flow chart for a method for analyzing the coronary vasculature according to an embodiment. In the exemplary embodiment of Fig. 4, the diagnostic image data 10 is received, in step S101, at the modeling unit 100 and segmented to generate the physiological model. In step S102, the modeling unit 100 generates the physiological model and provides the physiological model to the determination unit 200.

In the exemplary embodiment of Fig. 4, information regarding the candidate positions 32', 32" is provided to the determination unit 200. This information is determined by the input unit 300. Therefore, in this particular example, a subset of the steps performed by the input unit 300 are performed at the same time as the steps performed by the modeling unit 100. More particularly, in step S301, the input unit 300 receives the intravascular hemodynamic parameter value 20, the volumetric reconstruction 30 and the tracking image 40. In step S302, the input unit determines, based on a co-registration of the tracking image 40 and the volumetric reconstruction 30, the plurality of the candidate positions 32', 32" and provides them to the determination unit 200 in step S303.

In step S201, the physiological model is received at the determination unit 200 from the modeling unit 100. Optionally, in step S201, further the information regarding the candidate positions 32', 32" is received from the input unit 300. Subsequently, in step S202, the determination unit 200 determines a plurality of simulated hemodynamic parameter values. In the specific example of Fig. 4, where the determination unit 200 has received information regarding the candidate positions 32', 32", the determination unit 200 determines a simulated hemodynamic parameter value for each candidate position 32', 32". The plurality of the thus determined simulated hemodynamic parameter values is then, in this example along with the respective information about the corresponding candidate positions 32', 32", provided to the input unit 300.

In step S304, the input unit 300 receives the plurality of simulated hemodynamic parameters. In the exemplary embodiment according to Fig. 4, each of the simulated hemodynamic parameter values is received along with its respective candidate position 32', 32". In step S305, the input unit 300 provides the plurality of simulated hemodynamic parameters, optionally along with their respective candidate positions 32', 32", as well as the intravascular hemodynamic parameter value 20 and the volumetric reconstruction 30 to the comparing unit 400.

Comparing unit 400 compares, in step S401, each of the simulated hemodynamic parameter values to the intravascular hemodynamic parameter value 20. In step S402, the comparing unit 400 identifies the simulated hemodynamic parameter value that matches the intravascular hemodynamic parameter value 20 the best, thereby identifying the vessel of interest in the volumetric reconstruction 30. According to the exemplary embodiment of Fig. 4, the comparing unit 400 then provides an identification of the vessel of interest in the volumetric reconstruction 30 together with the volumetric reconstruction 30 and the intravascular hemodynamic parameter value 20 to the calculation unit 500 in step S403.

In step S501, the calculation unit 500 calculates a first representation of the intravascular hemodynamic parameter value 20. In step S502, the calculation unit 500 calculates a second representation of the volumetric reconstruction 30. In step S503, the calculation unit 500 provides first and second representation to the display unit 600. In step S601, the display unit 600 annotates the first graphical representation to the second graphical representation. In step S602, the display unit jointly displays the first and second graphical representation.

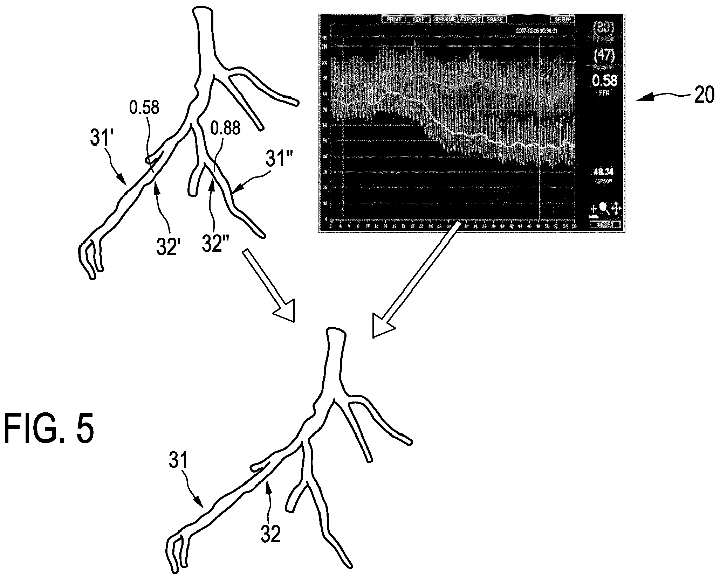

Fig. 5 schematically illustrates the principle of an identification of the vessel of interest in a volumetric reconstruction from two candidate vessels 31', 31" by comparing the simulated hemodynamic parameter value calculated for two candidate positions 32', 32" to an intravascular hemodynamic parameter value 20.

In the exemplary embodiment of Fig. 5, the hemodynamic parameter value corresponds to a value of the fractional flow reserve (FFR). As such, the simulated hemodynamic parameter value corresponds to a simulated FFR value and the intravascular hemodynamic parameter value 20 corresponds to an FFR value that has been acquired in-situ from the vessel of interest.

In Fig. 5, the first simulated hemodynamic parameter value at first candidate position 32' in first candidate vessel 31' has been calculated as 0.58. The second simulated hemodynamic parameter value at second candidate position 32" in second candidate vessel 31" has been calculated as 0.88. The intravascular hemodynamic parameter value 20 has been determined as 0.58. Thus, a comparison between first and second simulated hemodynamic parameter value with the intravascular hemodynamic parameter value 20, respectively, shows a higher agreement between first simulated hemodynamic parameter value with the intravascular hemodynamic parameter value 20. Thus, first candidate vessel 31' is determined to be the vessel of interest 31 in the volumetric reconstruction 30 and candidate position 32 in vessel of interest 31 is considered as corresponding to the intravascular position at which the in-situ acquisition of intravascular hemodynamic parameter value 20 has been performed.

Thus, in the exemplary embodiment, the co-registration of the invasive FFR measurement with the non-invasively acquired volumetric reconstruction may be improved such that the vessel of interest may be uniquely identified in the volumetric reconstruction of the vasculature.

Although in above described embodiments, the diagnostic image data has been obtained using computed tomography, it shall be understood that in other embodiments, the diagnostic image data may likewise be retrieved by other imaging methods, such as position emission tomography, single positron emission computed tomography, magnetic resonance imaging, X-ray scanning or the like.

Further, it shall be understood that, although in the above embodiments the values have been determined for a single hemodynamic parameter, the method may also be performed by determining the values for more than one hemodynamic parameter in measurement and by simulation, respectively. To that end, it may be understood that the hemodynamic parameter regarded may be a hemodynamic parameter other than blood pressure, such as blood resistance or blood viscosity or the like.

Further, while in the above embodiments, the analyzing has been performed on the coronary vasculature, in other embodiments, the analysis may likewise be performed on the vasculature in other parts of the human body, such as the peripheral vasculature.

It shall be understood that, while in the above embodiments the physiological model is representative of the fluid dynamics, i.e. comprises a fluid dynamics model, the physiological model may further be representative of the geometry of the vasculature, i.e. may further comprise a geometric model of the vasculature.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the segmenting of the diagnostic image data, the generating of a physiological model, the determination of the simulated hemodynamic parameter values, the comparing of the simulated and intravascular hemodynamic parameter values, the identifying of the vessel of interest, the calculation of a graphical representation, et cetera performed by one or several units or devices can be performed by any other number of units or devices. These procedures in accordance with the invention can hereby be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for analyzing a vasculature of a patient, comprising an input unit configured to receive a plurality of simulated hemodynamic parameters determined on the basis of a physiological model derived from diagnostic image data of the vasculature for a plurality of candidate vessels of the vasculature and at least one intravascular hemodynamic parameter acquired in-situ by an intravascular measurement device from a vessel of interest in the vasculature. The apparatus further comprises a comparing unit configured to compare each of the plurality of simulated hemodynamic parameters to the at least one intravascular hemodynamic parameter and to identify, based on the comparing, the vessel of interest in a reconstruction derived from the diagnostic image data.

By means of this apparatus, the ambiguity in the co-registration of the imaging modality with the intravascular measurement modality may be avoided.

## Claims

1. An apparatus for analyzing a vasculature of a patient, comprising
an input unit configured to receive
a plurality of simulated hemodynamic parameter values determined on the basis of a physiological model derived from diagnostic image data of the vasculature for a plurality of candidate vessels of the vasculature; and
at least one intravascular hemodynamic parameter value acquired in-situ by an intravascular measurement device from a vessel of interest in the vasculature;
and
a comparing unit configured to
compare each of the plurality of simulated hemodynamic parameter values to the at least one intravascular hemodynamic parameter value; and
identify, based on the comparing, the vessel of interest in a reconstruction derived from the diagnostic image data.

2. Apparatus according to claim 1, wherein
the diagnostic image data comprises computed tomography (CT) projection data; and the reconstruction comprises volumetric data reconstructed from the computed tomography projection data.

3. Apparatus according to claim 1, wherein
the at least one intravascular hemodynamic parameter value is acquired using the intravascular measurement device for pullback recording.

4. Apparatus according to claim 1, further comprising
a modeling unit configured to generate the physiological model based on the diagnostic image data of the vasculature, the physiological model representing the fluid dynamics through the plurality of candidate vessels; and
a determination unit configured to determine the plurality of simulated hemodynamic parameter values on the basis of the physiological model.

5. Apparatus according to claim 4, wherein
the determining the plurality of simulated hemodynamic parameter values comprises determining, for each of the candidate vessels, a respective simulated hemodynamic parameter value; and
the identifying the vessel of interest comprises identifying the candidate vessel for which the respective simulated hemodynamic parameter value exhibits the best agreement with the at least one intravascular hemodynamic parameter value.

6. Apparatus according to claim 1, wherein
the input unit is further configured to receive at least one additional information indicative of the vessel of interest; wherein
the comparing each of the plurality of simulated hemodynamic parameter values to the at least one intravascular hemodynamic parameter value comprises co-registering the at least one intravascular hemodynamic parameter value to the reconstruction of the diagnostic image data based on the at least one additional information; and
a deriving, based on the co-registering, the plurality of candidate vessels.

7. Apparatus according to claim 1, wherein
the input unit is further configured to receive at least one tracking image of the measurement device; and wherein
the comparing each of the plurality of simulated hemodynamic parameter values to the at least one intravascular hemodynamic parameter value comprises a co-registering the at least one tracking image to the reconstruction of the diagnostic image data; and
a deriving, based on the co-registering, the plurality of candidate vessels.

8. Apparatus according to claim 7, wherein
the measurement device is configured to acquire the at least one hemodynamic parameter value at a particular intravascular position in the vessel of interest;
the deriving the plurality of candidate vessels comprises identifying, for each of the candidate vessels, a respective candidate position corresponding to the intravascular position;
the determining the simulated hemodynamic parameter values comprises determining, for each of the candidate vessels, a respective simulated hemodynamic parameter value at the respective candidate position;
the comparing comprises comparing the at least one intravascular hemodynamic parameter value acquired at the intravascular position to each of the simulated hemodynamic parameter values determined at the respective candidate positions; and
the identifying the vessel of interest comprises identifying the candidate position for which the respective simulated hemodynamic parameter value exhibits the best agreement with the at least one intravascular hemodynamic parameter value.

9. Apparatus according to claim 1, further comprising
a calculation unit configured to calculate a graphical representation of the at least one intravascular hemodynamic parameter value and a second graphical representation of the reconstruction of the diagnostic image data; and
a display unit configured to jointly display the first and second representation.

10. Apparatus according to claim 9, wherein the jointly displaying the first graphical representation and the second graphical representation comprises an annotating the first graphical representation to the second graphical representation.

11. A method for analyzing a vasculature of a patient, the method comprising the steps of:
receiving a plurality of simulated hemodynamic parameter values determined on the basis of a physiological model derived from diagnostic image data of the vasculature for a plurality of candidate vessels of the vasculature;
receiving at least one intravascular hemodynamic parameter value acquired in-situ by an intravascular measurement device from a vessel of interest in the vasculature;
comparing each of the plurality of simulated hemodynamic parameter values to the at least one intravascular hemodynamic parameter value; and
identifying, based on the comparing, the vessel of interest in a reconstruction derived from the diagnostic image data.

12. Method according to claim 11, further comprising the steps of:
receiving at least one tracking image of the measurement device;
co-registering the at least one tracking image to the reconstruction of the diagnostic image data; and
deriving, based on the co-registering, the plurality of candidate vessels.

13. Method according to claim 11, further comprising the steps of:
calculating a graphical representation of the at least one intravascular hemodynamic parameter value and a second graphical representation of the reconstruction of the diagnostic image data; and
jointly displaying the first and second representation.

14. A computer program for controlling an apparatus according to anyone of claims 1 to 10, which, when executed by a processing unit, is adapted to perform the method according to anyone of claims 11 to 13.

15. A computer-readable medium having stored thereon the computer program according to claim 14.
